# EUROPÄISCHE PATENTSCHRIFT

(11) **EP 1 045 947 B1**
(45) Veröffentlichungstag und Bekanntmachung des Hinweises auf die Patenterteilung: **26.01.2005**
(21) Anmeldenummer: 99947309.3
(22) Anmeldetag: 09.09.1999
(51) Int. Cl.: E05B 49/00, B60R 25/00, H04B 13/00, H04B 5/00

(54) **ELEKTRONISCHES KOMMUNIKATIONSSYSTEM**
ELECTRONIC COMMUNICATIONS SYSTEM
SYSTEME DE COMMUNICATION ELECTRONIQUE

(30) Priorität: 14.09.1998 EP 98117343
(43) Veröffentlichungstag der Anmeldung: 25.10.2000
(73) Patentinhaber: Koninklijke Philips Electronics N.V., 5621 BA Eindhoven (NL); Philips Intellectual Property & Standards GmbH, 20099 Hamburg (DE)
(72) Erfinder: GIESLER, Thomas, NL-5621 BA Eindhoven (NL)
(74) Vertreter: Peters, Carl Heinrich, Dipl.-Ing.
(86) Internationale Anmeldenummer: PCT/EP1999/006696
(87) Internationale Veröffentlichungsnummer: WO 2000/015931

(56) Entgegenhaltungen:
- EP-A- 0 843 425
- WO-A-89/02507
- WO-A-96/36134
- GB-A- 2 290 435
- US-A- 5 204 672

## Beschreibung

Die Erfindung bezieht sich auf ein elektronisches Kommunikationssystem für ein Fahrzeug mit einer im Fahrzeug angeordneten Basisstation und wenigstens einem beweglichen Datenträger, der zum Austausch von Datensignalen mit der Basisstation eingerichtet ist, gemäß dem Oberbegriff des Anspruchs 1.

Derartige Kommunikationssysteme dienen insbesondere der Zugangskontrolle zum Fahrzeug. Derartige Systeme sind als schlüssellose Zentralverriegelungssysteme oder Wegfahrsperren bekannt geworden. Ein solches System ist in dem Aufsatz "Türsteher ohne Bodygard-Format" von Dr. Stephan Schmitz und Jacek Kruppa, erschienen in der Zeitschrift "Elektronik", Heft 22, 1998, Seiten 148 bis 156, beschrieben.

In diesem Aufsatz wird ein schlüsselloses Zentralverriegelungssystem für Fahrzeuganwendungen dargestellt, welches die folgenden Bestandteile umfaßt:
Einen Transponder, der in einer Chipkarte oder in einem Schlüsselkopf eingebettet werden kann und mit Antennenspule, Batterie, UHF-Sender und Drucktasten ausgerüstet sein kann,
eine NF-Antenne im Fahrertürspiegel,
einen UHF-Empfänger im Fahrzeug, der in Aktion tritt, sobald er, bewußt ausgelöst vom Benutzer, eine entsprechende UHF-Sequenz vom Transponder empfängt,
einen mechanischen Schalter im Türgriff zum Aktivieren eines passiven Zugangssystems mit einer zusätzlichen Drucktaste zum Initiieren des Verriegelungsvorgangs,
die Basisstation oder das Türmodul und ein Steuergerät.

Vom Transponder werden dabei Datensignale zum Fahrzeug über über UHF-Verbindung übertragen, wohingegen vom Fahrzeug zum Transponder in jedem Fall eine Übertragung durch ein NF-Signal mit einer Frequenz von 125 kHz erfolgt. Wahlweise kann auch die Übertragung vom Transponder zum Fahrzeug mit einem solchen NF-Signal erfolgen.

Ein derartiges schlüsselloses Verriegelungssystem ist in der Lage, das Öffnen eines Fahrzeugs im Vergleich zur Benutzung eines mechanischen Türschlüssels in gewissem Maße zu erleichtern. Allerdings ist diese Erleichterung dadurch begrenzt, daß der Benutzer nach wie vor den Transponder hervorsuchen und bedienen muß. Damit ist das Problem ungelöst, den Transponder stets in einer Tasche oder dergleichen bei sich führen zu müssen und zur Bedienung aus dieser zu entnehmen. Im übrigen gilt dieser geringfügige Handhabungsvorteil auch nicht mehr für die Funktion der Wegfahrsperre, d.h. für das Starten des Fahrzeugs.

Die Erfindung hat die Aufgabe, ein elektronisches Kommunikationssystem zu schaffen, durch welches unter den unterschiedlichsten Anwendungsbedingungen eine sehr einfache Handhabung gewährleistet ist.

Erfindungsgemäß wird diese Aufgabe bei einem elektronischen Kommunikationssystem der eingangs genannten Art durch die Merkmale des Anspruchs 1 gelöst.

Das erfindungsgemäße Kommunikationssystem ermöglicht unter verschiedensten Anwendungsbedingungen außer einer sehr einfachen Handhabung auch ein Höchstmaß an Betätigungssicherheit, insbesondere ein Höchstmaß an Schutz gegen unerlaubten Zugriff. Dies wird insbesondere dadurch erreicht, daß die erfindungsgemäß ausgebildeten Koppelstrecken eine Reichweite aufweisen, die auf das für eine bedienerfreundliche Handhabung erforderliche Minimum begrenzt ist und somit einen unerlaubten Zugriff durch nicht autorisierte Dritte ausschließt. Dies wird insbesondere durch die kapazitiven Verbindungen erreicht. Außerdem ist das erfindungsgemäße Kommunikationssystem derart variabel gestaltet, daß damit verschiedene Anwendungen und Bedienungsarten einfach realisiert werden können. Insbesondere ist eine Unterscheidung der Bedienung von einem Ort außerhalb oder innerhalb des Fahrzeuges nicht erforderlich.

In einer vorteilhaften Weiterbildung des erfindungsgemäßen Kommunikationssystems ist die erste Koppelstrecke wenigstens teilweise durch den Verschiebungsströme führenden Körper eines Benutzers gebildet. Dies hat zum einen den Vorteil, daß eine derart ausgebildete Koppelstrecke besonders abhörsicher gestaltet sein kann. Zum anderen wird dadurch eine besonders einfache Ausgestaltung und Handhabung des Kommunikationssystems erreicht. Nach einer anderen Ausbildung der Erfindung ist die zweite Koppelstrecke wenigstens teilweise durch den Boden gebildet. Bei einem Landfahrzeug ist dies der Erdboden bzw. die Fahrbahn. Für ein Wasserfahrzeug kann die zweite Koppelstrecke auch wenigstens teilweise durch das Gewässer gebildet sein. Auch diese Ausbildung dient der Einfachheit des Kommunikationssystems; insbesondere wird unabhängig von der Position des Fahrzeugs der Boden stets eine Verbindung zwischen dem Fahrzeug und einem sich außerhalb des Fahrzeugs befindlichen Benutzer bilden.

Vorzugsweise enthält das erfindungsgemäße Kommunikationssystem wenigstens eine zusätzliche Daten- und/oder Energieübertragungsstrecke, die eine im wesentlichen magnetische Kopplung zwischen dem (den) Datenträger(n) und der Basisstation beinhaltet. Durch diese zusätzliche Einrichtung wird ein Rückfallsystem geschaffen, welches zwar in seiner Bedienung und seiner Sicherheit nur geringeren Anforderungen genügt, jedoch bei Ausfall der beschriebenen Einrichtungen des erfindungsgemäßen Kommunikationssystems eine Notfallfunktion ermöglicht.

Wahlweise kann dabei anstelle der magnetischen Kopplung auch eine Kopplung durch elektromagnetische Wellen im UHF-Bereich oder durch infrarotes Licht erfolgen.

Durch die Erfindung wird somit ein elektronisches Kommunikationssystem bereitgestellt, welches auf aktiv kapazitiv gekoppelten Datenträgem und vorzugsweise innerhalb des Körpers eines Benutzers geführten elektrischen Feldern beruht. Ein vorzugsweise mit induktiver Kopplung ausgebildetes Notsystem kann integriert werden. Mit diesem Kommunikationssystem ist die Bedienung eines Fahrzeugs in der Weise möglich, daß der Benutzer ohne gesonderte Betätigung eines Schlüssels oder vergleichbaren Zugangskontrollsystems allein durch Betätigung eines Türgriffs Zugang zum Fahrzeug erhält und in ebenso einfacher Weise zum Beispiel durch Betätigen einer Startertaste das Fahrzeug in Betrieb setzen kann. Das erfindungsgemäße elektronische Kommunikationssystem überwacht dabei die Zugangsberechtigung und bildet die erforderlichen Sicherungsmaßnahmen gegen unbefugte Betätigungen. Zum Erfüllen dieser Aufgabe sind vom Benutzer keinerlei zusätzliche Handlungen wie beispielsweise die Betätigung eines Femsteuersenders oder eines mechanischen Schlüssels erforderlich. Der zugangsberechtigte Benutzer wird vielmehr vollautomatisch durch das erfindungsgemäße Kommunikationssystem erkannt und autorisiert. Der dazu gehörige, bewegliche Datenträger wird vom Benutzer in bzw. unter seiner Kleidung oder auch in einer Handtasche oder dergleichen mit geführt. Die Elektroden können in einfacher Weise durch Annäherungs- bzw. Berührungsfelder in den Betätigungselementen des Fahrzeugs, beispielsweise dem Türgriff oder einer Startertaste, gebildet sein. Durch die erfindungsgemäße Anordnung dieser Elektroden im Kommunikationssystem wird ein Höchstmaß an Bedienungskomfort und Zugriffssicherheit, insbesondere Sicherheit gegen unerlaubten Zugriff, erreicht.

An dieser Stelle sei bemerkt, daß aus der Druckschrift WO 96/36134 ein drahtloses System bekannt ist, welches einen Sender und einen Empfänger enthält, die durch einen Benutzer und das Massepotential eines Raumes gekoppelt sind. Der Sender erzeugt niederfrequente Signale niedriger Leistung, die durch kapazitive Kopplung als Verschiebungsströme durch den Körper des Benutzers fließen. Das verteilte Massepotential des Raumes stellt den Rückflußpfad für den Strom dar.

Ferner sei bemerkt, daß aus der EP 0 843 425 A2 ein elektronisches Kommunikationsgerät bekannt ist, welches den menschlichen Körper als Übertragungsmedium benutzt. Dieses Gerät dient zum Verschlüsseln und Übertragen von Daten aus einem Sender, der vorzugsweise als Karte ausgebildet sein kann, an einen Empfänger, der vorzugsweise in einer Basisstation enthalten ist. Der Sender enthält einen Generator für ein elektrisches Feld, eine Datenverschlüsselungseinrichtung, die durch Modulation des elektrischen Feldes tätig wird und Elektroden, um das elektrische Feld durch den menschlichen Körper zu koppeln. Der Empfänger enthält Elektroden, die in physischem Kontakt oder dichter Annäherung zu einem Teil des menschlichen Körpers stehen, um ein elektrisches Feld zu detektieren, welches durch den Körper übertragen wird. Ein Demodulator im Empfänger extrahiert die Daten aus dem modulierten elektrischen Feld. Es ist ferner angegeben, daß eine Empfängerelektrode in einem metallischen Türgriff eines Kraftfahrzeugs angeordnet sein kann. Die Türen sollen dann automatisch entriegelt werden, wenn der Besitzer einer autorisierten Karte, d.h. eines autorisierten Senders, mit der Hand den Türgriff berührt. Eine Berührung des Türgriffs ohne Ziehen desselben über eine gewisse Zeit, beispielsweise 15 Sekunden, soll ein Verriegeln aller Türen bewirken.

Aus diesen Druckschriften ist zwar grundsätzlich bekannt, elektrische Felder zur Datenübertragung mittels Verschiebungsströmen durch den menschlichen Körper einzusetzen. Diese Schriften geben jedoch keine Lehre darüber, wie ein solches System im Sinne der Lösung der vorliegenden Aufgabenstellung auszugestalten ist.

Ausführungsbeispiele der Erfindung sind in der Zeichnung dargestellt und werden im nachfolgenden näher beschrieben. In den Zeichnungen, in denen übereinstimmende Elemente mit denselben Bezugszeichen versehen sind, zeigen
Fig. 1 eine schematische Darstellung eines ersten Ausführungsbeispiels des erfindungsgemäßen, elektronischen Kommunikationssystems,
Fig. 2 eine schematische Darstellung eines ersten Betriebsfalles des Kommunikationssystems nach Fig. 1,
Fig. 3 eine schematische Darstellung eines zweiten Betriebsfalles des Kommunikationssystems nach Fig. 1,
Fig. 4 eine schematische Darstellung eines dritten Betriebsfalles des Kommunikationssystems nach Fig. 1,
Fig. 5 eine schematische Darstellung eines vierten Betriebsfalles des Kommunikationssystems nach Fig. 1 und
Fig. 6 eine blockschematische Darstellung eines Ausführungsbeispiels für eine Basisstation und einen Datenträger des Kommunikationssystems nach Fig. 1.

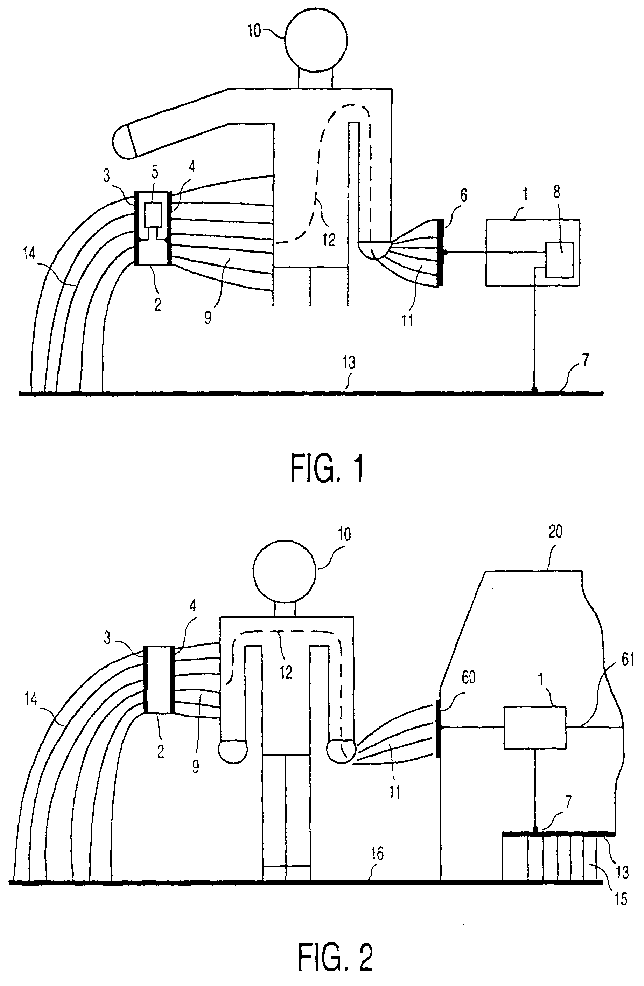

Fig. 1 zeigt ein Ausführungsbeispiel eines erfindungsgemäßen elektronischen Kommunikationssystems, in welchem der Austausch von Datensignalen wenigstens teilweise über den Körper eines Benutzers und gleichzeitig oder wahlweise durch den Boden vorgenommen werden kann. Darüber hinaus kann das dargestellte Beispiel des erfindungsgemäßen Kommunikationssystems auch ohne Einbindung dieser beiden Übertragungsmedien ausgeformt sein.

In der Prinzipdarstellung der Fig. 1, die zugleich einen ersten Betriebsfall des Ausführungsbeispiels zeigt, umfaßt das elektronische Kommunikationssystem eine Basisstation 1, die in einem Fahrzeug angeordnet ist, und wenigstens einen beweglichen Datenträger 2, der zum Austausch von Datensignalen mit der Basisstation 1 eingerichtet ist. Der Datenträger 2 weist eine erste Elektrode 3 und eine zweite Elektrode 4 auf. Bevorzugt ist der Datenträger 2 im wesentlichen flächig ausgebildet, beispielsweise in Kartenform. Die Elektroden 3, 4 bedecken dann wenigstens teilweise die einander gegenüberliegenden Hauptflächen der Karte. Eine erste Datensignalverarbeitungsschaltung 5, die zum Empfangen und/oder Senden der Datensignale von bzw. zu der Basisstation 1 ausgebildet ist, ist vom Datenträger 2 umfaßt. Die Datensignale werden dabei durch eine Spannung zwischen der ersten Elektrode 3 und der zweiten Elektrode 4 gebildet; der Datenträger 2 enthält entsprechende Verbindungen zwischen der ersten Datensignal-Verarbeitungsschaltung 5 und den Elektroden 3, 4.

Die Basisstation 1 weist wenigstens eine dritte Elektrode 6 und eine vierte Elektrode 7 sowie eine zweite Datensignal-Verarbeitungsschaltung 8 auf. Die zweite Datensignal-Verarbeitungsschaltung 8 ist zum Empfangen und/oder Senden der Datensignale von bzw. zu dem Datenträger 2 ausgebildet. Auch hier werden die Datensignale durch eine Spannung zwischen der dritten und der vierten Elektrode 6 bzw. 7 gebildet; entsprechende Verbindungen sind zwischen der zweiten Datensignal-Verarbeitungsschaltung 8 und der dritten bzw. vierten Elektrode 6 bzw, 7 angeordnet.

Im Betrieb des vorliegenden Ausführungsbeispiels des erfindungsgemäßen Kommunikationssystems sind die zweite Elektrode 4 und die dritte Elektrode 6 über eine erste Koppelstrecke zur Übertragung der Datensignale miteinander gekoppelt. Gemäß Fig. 1 umfaßt diese erste Koppelstrecke eine erste kapazitive Verbindung 9 über ein elektrisches Feld zwischen der zweiten Elektrode 4 und dem Körper eines Benutzers 10. Eine zweite kapazitive Verbindung 11 in der ersten Koppelstrecke wird über ein elektrisches Feld zwischen dem Körper des Benutzers 10, hier insbesondere seiner Hand, und der dritten Elektrode 6 gebildet. Außerdem ist gemäß Fig. 1 die erste Koppelstrecke wenigstens teilweise durch den Verschiebungsströme 12 führenden Körper des Benutzers 10 gebildet. Die erste und die zweite kapazitive Verbindung 9, 11 sowie der Körper des Benutzers 10 mit den darin geführten Verschiebungsströmen 12 sind zur Bildung der ersten Koppelstrecke in Reihe geschaltet.

In Fig. 1 ist weiterhin die vierte Elektrode 7 mit dem elektrischen Massekörper 13 des Fahrzeugs elektrisch verbunden. Zwischen dem elektrischen Massekörper 13 und der ersten Elektrode 3 besteht im Betrieb des dargestellten Kommunikationssystems eine Kopplung über eine zweite Koppelstrecke zur Übertragung der Datensignale. In der einfachsten Darstellung der Fig. 1 umfaßt die zweite Koppelstrecke eine dritte kapazitive Verbindung 14 über ein elektrisches Feld zwischen der ersten Elektrode 3 und dem Massekörper 13.

Variationen und unterschiedliche Betriebsfälle des Ausführungsbeispiels des Kommunikationssystems gemäß Fig. 1 sind in den folgenden Figuren dargestellt. Dabei kann die Basisstation 1 mit mehreren Elektroden verbunden sein, die jede für sich die Funktion der dritten Elektrode 6 ausüben. Das heißt, daß über jede dieser Elektroden wahlweise die erste Koppelstrecke geführt werden kann. Vorzugsweise sind diese Elektroden für die unterschiedlichen Bedienungszwecke an unterschiedlichen Stellen außerhalb und innerhalb des Fahrzeugs angebracht.

Fig. 2 zeigt ein Ausführungsbeispiel des erfindungsgemäßen Kommunikationssystems, bei dem sich der Benutzer 10 außerhalb des Fahrzeugs 20 befindet. Eine derartige Konfiguration des erfindungsgemäßen Kommunikationssystems ergibt sich insbesondere für die Ausübung der Zutrittskontrolle zum Fahrzeug, d.h. für die Verriegelung bzw. autorisierte Entriegelung der Türen oder auch einer Kofferraumklappe. Dazu befindet sich eine dritte Elektrode 60 an ihrem ersten Anbringungsort am Türgriff oder an dem Betätigungselement für die Kofferraumklappe an der Fahrzeugaußenhaut. Die Verbindungen und insbesondere Koppelstrecken für die Übertragung der Datensignale werden nun wie folgt gebildet. Die erste Koppelstrecke umfaßt wiederum die erste kapazitive Verbindung 9 zwischen der zweiten Elektrode 4 des Datenträgers 2 und dem Körper des Benutzers 10. Über Verschiebungsströme 12 im Körper des Benutzers 10 werden die Datensignale bis in die Hand des Benutzers 10 geleitet. Von der Hand des Benutzers 10 besteht die zweite kapazitive Verbindung 11 zur dritten Elektrode 60 am Türgriff. Die zweite Koppelstrecke gemäß Fig. 2 umfaßt zunächst die dritte kapazitive Verbindung 14 zwischen der ersten Elektrode 3 des Datenträgers 2 und dem Boden, d.h. dem Erdboden oder der Fahrbahn, auf der sich das Fahrzeug 20 befindet. Dieser Boden sit mit dem Bezugszeichen 16 versehen. Eine vierte kapazitive Verbindung 15 besteht ferner zwischen dem Boden 16 und dem elektrischen Massekörper 13 des Fahrzeugs 20. Innerhalb des Bodens 16 kann die Verbindung wieder bevorzugt durch Verschiebungsströme erfolgen. Auch in der zweiten Koppelstrecke sind die beiden kapazitiven Verbindungen 14, 15 und der Boden 16 bzw. die darin fließenden Verschiebungsströme in Reihe geschaltet.

In Fig. 2 ist neben der Verbindung zwischen der dritten Elektrode 60 und der Basisstation 1 auch eine weitere Verbindung 61 angedeutet, die zu einer weiteren dritten Elektrode führt. Anstelle der weiteren Verbindung 61 können auch mehrere solche weitere Verbindungen treten, je nach Anzahl der im bzw. am Fahrzeug 20 angeordneten dritten Elektroden.

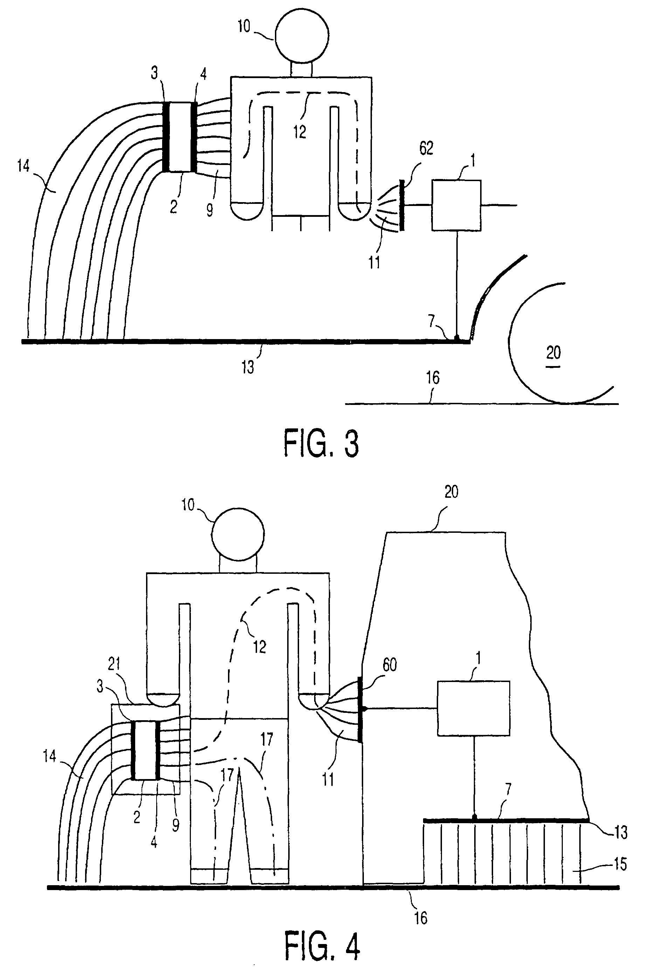

Fig. 3 zeigt eine Variation des Ausführungsbeispiels des erfindungsgemäßen Kommunikationssystems, bei der sich der Benutzer 10 innerhalb des Fahrzeugs befindet. Diese Variation kommt bevorzugt zur Ausführung für den Fall, in dem ein autorisierter Startvorgang für den Motor des Fahrzeugs 20 vorzunehmen ist. Auch bei dieser Variation besteht die erste Koppelstrecke aus der ersten kapazitiven Verbindung 9 und der zweiten kapazitiven Verbindung 11 sowie den Verschiebungsströmen 12 im Körper des Benutzers 10. Im Gegensatz zur allgemeinen Darstellung der Fig. 1 ist in Fig. 3 die erste kapazitive Verbindung 9 in einer Weise dargestellt, die eine besonders enge räumliche Nähe zwischen dem Benutzer 10 und dem Datenträger 2, d.h. eine besonders enge kapazitive Kopplung, symbolisieren soll. Wie noch gezeigt werden wird, ist dies jedoch für die Ausbildung des erfindungsgemäßen Kommunikationssystems nicht zwingend erforderlich. Die zweite kapazitive Verbindung 11 führt in Fig. 3 auf eine dritte Elektrode 62, die an einem zweiten Anbringungsort angeordnet ist, der sich vorzugsweise am Armaturenbrett, insbesondere im Bereich eines Betätigungselements für den Starter des Fahrzeugmotors befindet. Vorzugsweise kann die dritte Elektrode 62 mit diesem Betätigungselement für den Starter kombiniert sein. Beim Betätigen durch die Hand des Benutzers 10 wird dann automatisch die Berechtigungskontrolle durchgeführt und bei deren positivem Ausgang der Motor des Fahrzeugs 20 gestartet.

Beim Ausführungsbeispiel nach Fig. 3 besteht die zweite Koppelstrecke aus der dritten kapazitiven Verbindung 14 zwischen der ersten Elektrode 3 und dem Massekörper 13 des Fahrzeugs 20. Von dort besteht eine unmittelbare, elektrisch leitende Verbindung über die vierte Elektrode 7 an die Basisstation 1.

Da an die Basisstation 1 sowohl die dritte Elektrode 60 am Anbringungsort "Türgriff" als auch die dritte Elektrode 62 am Anbringungsort "Betätigungselement für Starter" angeschlossen ist, läßt sich das so gebildete Kommunikationssystem sehr einfach und flexibel sowohl für die Zugangskontrolle zum Fahrzeug 20 als auch für die Autorisierung des Startvorgangs einsetzen. Das Kommunikationssystem ist somit sowohl für eine passive Zugangskontrolle als auch für eine passive Bewegungskontrolle, d.h. als Wegfahrsperre, einsetzbar. Die Bedienung ist dabei sehr einfach, da der Benutzer 10 lediglich den Türgriff und das Betätigungselement für den Starter handhaben muß. Eine gesonderte Betätigung beispielsweise durch einen Schlüssel entfällt. Durch die Anordnung der dritten Elektroden 60 bzw. 62 und das Mitführen des Datenträgers 2 werden die notwendigen Vorgänge des Datenaustausches, d.h. wird die Übertragung der erforderlichen Datensignale für die Berechtigungskontrollen automatisch ohne Zutun des Benutzers 10 durchgeführt. Einem unberechtigten Benutzer bleiben dabei zuverlässig die genannten Tätigkeiten verwehrt. Durch die kapazitive Kopplung ist bei dem erfindungsgemäßen Kommunikationssystem außerdem die Reichweite der Datenübertragung auf das Minimum eingeschränkt, so daß ein unberechtigter Dritter den Austausch von Datensignalen zwischen dem Datenträger 2 und der Basisstation 1 nicht abhören kann.

In den dargestellten Ausführungsbeispielen ist der Datenträger 2 als vorzugsweise kartenförmiges Element angegeben. Der Datenträger 2 kann jedoch auch die Form eines Schlüsselanhängers, einer Armbanduhr oder eines Uhrenarmbandes bzw. eines Teiles davon aufweisen. Daneben ist jede weitere andere Ausbildung möglich, beispielsweise als Kleideretikett oder dergleichen.

Das erfindungsgemäße Kommunikationssystem erlaubt darüber hinaus auch ohne zusätzlichen Aufwand die Identifizierung des Standortes des Benutzers 10. Diese Identifizierung ist über die Ausbildung der Koppelstrecken über die unterschiedlichen Anbringungsorte der dritten Elektroden 60, 62 sehr einfach möglich. Auch diese Identifizierung dient der Bedienungssicherheit des Kommunikationssystems. Falls beispielsweise der Benutzer 10 mit dem Datenträger 2 sich außerhalb des Fahrzeugs 20 befindet, kann sicher ausgeschlossen werden, daß eine zweite, nicht autorisierte Person, beispielsweise ein Kind, im Fahrzeug 20 eine erfolgreiche Betätigung der Bedienungselemente des Fahrzeugs 20 durchführt, sofern diese in die Zugriffskontrolle durch das erfindungsgemäße Kommunikationssystem eingebunden sind. Insbesondere kann eine solche Person nicht erfolgreich das Betätigungselement für den Starter auslösen. In einem anderen Fall kann durch die Erfindung in einfacher Weise ausgeschlossen werden, daß das Fahrzeug 20 von einem nicht autorisierten Benutzer von außen verschlossen werden kann, so lange sich der autorisierte Benutzer 10 innerhalb des Fahrzeugs aufhält.

Die Identifikation, von welcher der dritten Elektroden 60, 62 ein Betätigungsvorgang ausgelöst werden soll, kann in einfacher Weise durch eine Detektion erfolgen, über welche der Elektroden die Datenübertragung eingeleitet wird, d.h. wo die kapazitive Verbindung hergestellt wird. Außerdem kann eine logische Verknüpfung mit den genannten Betätigungselementen für Türgriff oder Starter erfolgen, die der Basisstation 1 eine entsprechende Information liefert. Durch solch eine logische Verknüpfung können umgekehrt auch alle gerade nicht ausgewählten dritten Elektroden deaktiviert werden. Die Bedienungsperson des Benutzers 10 ist somit stets exakt festlegbar. Zur Detektion, über welche der dritten Elektroden der Datenaustausch stattfinden soll, kann von der Basisstation 1 auch eine zyklische Abfrage aller dritten Elektroden auf Vorhandensein eines Datensignals, d.h. eines elektrischen Feldes, bzw. einer kapazitiven Verbindung erfolgen. Bei positivem Ergebnis einer solchen Abfrage wird dann automatisch der Datenaustausch für die Zugangs- bzw. Berechtigungskontrolle gestartet.

Die dritten Elektroden 60, 62 können bevorzugt als vom Fahrzeug 20 elektrisch isolierte Betätigungselemente ausgebildet sein. Ist dies konstruktiv nicht wünschenswert oder möglich, können an diesen Betätigungselementen elektrisch isolierte Elektrodenflächen angeordnet werden. Beispielsweise können solche Elektrodenflächen auf bzw. unter den Türgriffen angeordnet werden. Eine Anbringung beispielsweise unterhalb des Türgriffes bietet zugleich einen Witterungsschutz.

Auch eine Anbringung von derartigen Elektroden in den Fensterscheiben des Fahrzeugs 20 oder innerhalb eines Rückspiegels kann vorteilhaft sein.

Im Gegensatz zu diesen, für die Zugangskontrolle zum Fahrzeug 20 vorteilhaften Ausbildungen, kann die dritte Elektrode 62 für die Wegfahrsperre innerhalb des Fahrzeugs 20 vorzugsweise direkt auf dem Betätigungselement für den Starter angeordnet sein, beispielsweise auf der Oberfläche eines Tastschalters. Auch eine Ausbildung als durch Drehbewegung zu betätigenden, schlüsselartigen Schalters ist möglich, wobei die dritte Elektrode 62 auf der Oberfläche des Griffteils eines solchen Schalters angeordnet werden kann. Eine derartige Ausbildung könnte erwünscht sein, um dem Benutzer 10 die von konventionell ausgerüsteten Fahrzeugen übliche Betätigungsbewegung zu erhalten.

Alternativ kann die dritte Elektrode 62 durch das Lenkrad oder einen Teil davon gebildet sein. Auch eine Kombination mit einer entsprechenden Ausbildung des Betätigungselements für den Starter ist möglich. Der Startvorgang des Fahrzeugs wird dann durch Berühren des Lenkrades und gleichzeitiges Betätigen des Starters ausgelöst. Auch die Betätigung eines edals oder die kombinierte Betätigung mehrerer Pedale kann für die Auslösung des Startvorgangs in Kombination mit der Berührung der dritten Elektrode 62 herangezogen werden. Darüber hinaus kann die Sicherheit für eine exakte Identifizierung der Position des Benutzers 10 dadurch erhöht werden, daß zusätzliche dritte Elektroden auf wenigstens einem der Pedale angeordnet werden. In diesem Fall wird die Zugriffskontrolle nacheinander zweimal ausgeführt, das erste Mal über das Betätigungselement für den Starter, das zweite Mal über die Pedale, oder auch in umgekehrter Reihenfolge.

Die vorstehenden Überlegungen gelten uneingeschränkt für den Fall, daß der Datenträger 2 dicht am Körper des Benutzers 10 getragen wird, so daß die erste kapazitive Verbindung 9 stets sehr wirksam ist. Unterschiede in der Wirksamkeit des Kommunikationssystems zwischen der Funktion der Zugangskontrolle und der Funktion der Wegfahrsperre bestehen dann nicht.

Diese Verhältnisse verändern sich, falls es aus verschiedenen Gründen nicht angebracht ist, den Datenträge 2 dicht am Körper des Benutzers 10 zu tragen. Beispielsweise fehlt Damenbekleidung oft eine geeignete Tasche. Das erfindungsgemäße Kommunikationssystem ist nun derart beschaffen, daß der Datenträger ohne Einbuße der Funktionsfähigkeit auch in einer Handtasche, einem Aktenkoffer oder dergleichen mit geführt werden kann. Allerdings sollte ein derartiger Aktenkoffer keine stark elektrisch abschirmende Wirkung wie beispielsweise ein Aluminiumkoffer haben. Die elektrischen Feldstärken insbesondere für die erste kapazitive Verbindung 9 sind daher so dimensioniert, daß auch für das Tragen des Datenträgers zwei in einer Handtasche oder einem vergleichbaren Behältnis in relativer Nähe zum Körper noch eine einwandfreie Datenübertragung gewährleistet ist.

Derartige Verhältnisse sind beispielhaft in Fig. 4 dargestellt. Der Benutzer 10 trägt hier den Datenträger 2 in einem Aktenkoffer 21 bei sich. Die erste kapazitive Verbindung 9 führt dann von der zweiten Elektrode 4 zum Körper des Benutzers 10 und setzt sich in diesem durch die Verschiebungsströme 12 fort.

Bei der Konfiguration nach Fig. 4 können jedoch außer einem vergrößerten Abstand zwischen der zweiten Elektrode 4 und dem Körper des Benutzers 10 auch parasitäre Verschiebungsströme 17 auftreten, die hier durch die Beine des Benutzers 10 zum Boden 16 fließen und über diesen eine Verbindung zur dritten kapazitiven Verbindung 14 schaffen. Durch die Dimensionierung der Elemente des Datenträgers 2, insbesondere der ersten Datensignal-Verarbeitungsschaltung 5, ist sicherzustellen, daß auch diese parasitären Verschiebungsströme eine korrekte Übertragung der Datensignale nicht gefährden.

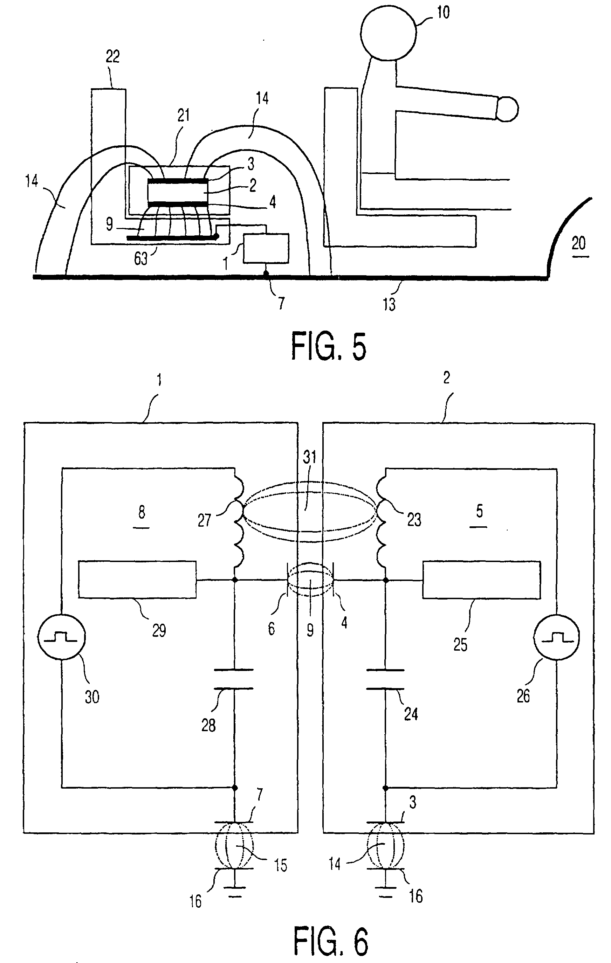

Eine veränderte Konfiguration des erfindungsgemäßen Kommunikationssystems ergibt sich jedoch, wenn ein nicht unmittelbar am Körper des Benutzers 10 mitgeführter Datenträger im Fahrzeug 20 abgelegt wird. Hier kann der Fall eintreten, daß die räumliche Entfernung zwischen dem Datenträger, insbesondere dessen zweiter Elektrode 4, und dem Körper des Benutzers 10 für eine fehlerfreie Datenübertragung zu groß wird. Fig. 5 zeigt derartige Verhältnisse am Beispiel eines Datenträgers 2, der in einem Aktenkoffer 21 auf einem Fahrzeugsitz 22 abgelegt ist. Eine Datenübertragung über den Körper des Benutzers 10 erfolgt nun nicht mehr. Statt dessen sind im Fahrzeug an weiteren Anbringungsorten weitere dritte Elektroden angebracht. Als Beispiel dafür zeigt Fig. 5 eine dritte Elektrode 63 am dritten Anbringungsort in der Sitzfläche des Fahrzeugsitzes 22. Die dritte Elektrode 63 kann durch ein metallisches Geflecht oder Metalldrähte in den Sitzflächen oder durch die metallischen Sitzfedern gebildet sein, auch eine Sitzheizung durch elektrische Heizdrähte kann hierfür herangezogen werden. Die dritte Elektrode 63 ist in gleicher Weise wie die dritten Elektroden 6, 60 bzw. 62 mit der Basisstation 1 verbunden und kann über diese Verbindung auch in gleicher Weise identifiziert werden. Die erste Koppelstrecke umfaßt bei dieser Konfiguration lediglich die erste kapazitive Verbindung 9, die zweite Koppelstrecke besteht lediglich aus der dritten kapazitiven Verbindung 14. Auf diese Weise ist auch in dieser Betriebssituation eine sehr funktionssichere und einfache Datenübertragung mit dem erfindungsgemäßen Kommunikationssystem möglich.

Fig. 6 zeigt ein Ausführungsbeispiel für den grundsätzlichen Schaltungsaufbau des Datenträgers 2 und der Basisstation 1. In diesem Ausführungsbeispiel umfaßt die erste Datensignal-Verarbeitungsschaltung 5 des Datenträgers 2 einen Resonanzkreis, der eine Induktivität 23 und eine Kapazität 24 enthält sowie einen Schaltkreis mit einem Demodulator. Dieser Schaltkreis 25 dient der Demodulation empfangener Datensignale und ihrer Verarbeitung. Ferner ist eine Treiberschaltung 26 vorhanden zum Senden von Datensignalen. Die Induktivität 23 und die Kapazität 24 bilden einen Serienresonanzkreis, der an die Treiberschaltung 26 angeschlossen ist. Am Verbindungspunkt zwischen der Induktivität 23 und der Kapazität 24 sind außerdem die zweite Elektrode 4 und der Schaltkreis 25 angeschlossen. An den Verbindungspunkt zwischen der Kapazität 24 und der Treiberschaltung 26 ist die erste Elektrode 3 angeschlossen.

In vergleichbarer Weise umfaßt die zweite Datensignal-Verarbeitungsschaltung 8 der Basisstation 1 eine Induktivität 27, eine Kapazität 28, einen Schaltkreis 29 mit einem Demodulator und eine Treiberschaltung 30. Diese Elemente sind wie die vergleichbaren Elemente des Datenträgers 2 miteinander verbunden. Am Verbindungspunkt zwischen der Induktivität 27 und der Kapazität 28 ist die dritte Elektrode angeschlossen, am Verbindungspunkt zwischen der Kapazität 28 und der Treiberschaltung 30 ist die vierte Elektrode 7 angeschlossen. Während die erste, zweite, dritte und vierte Elektrode in der bereits beschriebenen Weise über die kapazitiven Verbindungen 9, 14, 15 miteinander verkoppelt sind, ist eine zusätzliche Kopplung zwischen den Induktivitäten 23 und 27 gegeben, die mit dem Bezugszeichen 31 versehen ist. Über diese zusätzliche, induktive Kopplung 31 können der Datenträger 1 und die Basisstation 2 ebenfalls Datensignale austauschen. In der Empfangsbetriebsart werden die Treiberschaltungen 26 bzw. 30 kurzgeschlossen und die Induktivitäten 23 bzw. 27 bilden mit den zugehörigen Kapazitäten 24 bzw. 28 Paralleiresonanzkreise.

Durch den gezeigten Aufbau kann sehr einfach ein Kommunikationssystem mit kapazitiven Verbindungen geschaffen werden, in welches eine induktive Verbindung integriert ist. Damit ist mit einfachen Mitteln eine Kommunikation wahlweise über beide Verbindungen möglich. Da die induktive Verbindung vorzugsweise nur als Notfallverbindung vorgesehen ist, können die zugehörigen Induktivitäten kleine räumliche Abmessungen aufweisen, und die induktive Kopplung kann so bemessen sein, daß sie nur über sehr geringe Entfernungen wirksam ist. Dies reicht für die genannte Notfallfunktion aus und erhöht die Abhörsicherheit des Kommunikationssystems.

Die Induktivitäten für die induktive Verbindung - auch als Antennenspulen bezeichnet - können auf seiten der Basisstation beispielsweise im Rückspiegel des Fahrzeugs oder im Armaturenbrett untergebracht werden. Eine Bedienung des Kommunikationssystems ist dann sowohl über die kapazitiven Verbindungen als auch über die induktiven Verbindungen von allen gewünschten Bedienungsorten aus möglich. Dies erhöht die Zuverlässigkeit des erfindungsgemäßen Kommunikationssystems durch eine allseits verfügbare Notfallfunktion, insbesondere auch bei ungünstigen Witterungsbedingungen.

Das erfindungsgemäße Kommunikationssystem ist in diesem Sinne besonders ausfallsicher, auch für den Fall, daß die Energieversorgung für die erste Datensignal-Verarbeitungsschaltung 5 des Datenträgers 2 vollständig ausfällt. Das erfindungsgemäße Kommunikationssystem ist darüber hinaus auch mit UHF- oder Infrarot-Datenverbindungen kombinierbar. Dies erhöht nicht nur die Betriebssicherheit durch Bereitstellen zusätzlicher Notfallfunktionen, sondern ermöglicht auch einen sehr universellen Einsatz in bereits bestehenden Systemen, welche sich derartiger Datenverbindungen bedienen.

## Patentansprüche

1. Elektronisches Kommunikationssystem mit einer Basisstation und wenigstens einem beweglichen Datenträger, der zum Austausch von Datensignalen mit der Basisstation eingerichtet ist, worin
- der Datenträger eine erste und eine zweite Elektrode sowie eine erste Datensignal-Verarbeitungsschaltung aufweist, die zum Empfangen und/oder Senden der Datensignale von bzw. zu der Basisstation ausgebildet ist, wobei die Datensignale durch eine Spannung zwischen der ersten und der zweiten Elektrode gebildet werden,
- die Basisstation wenigstens eine dritte und eine vierte Elektrode sowie eine zweite Datensignal-Verarbeitungsschaltung aufweist, die zum Empfangen und/oder Senden der Datensignale von bzw. zu dem (den) Datenträger(n) ausgebildet ist, wobei die Datensignale durch eine Spannung zwischen der dritten und der vierten Elektrode gebildet werden,
- die zweite und die dritte Elektrode im Betrieb über eine erste Koppelstrecke zur Übertragung der Datensignale miteinander gekoppelt sind,
- die erste Elektrode mit einem elektrischen Massekörper im Betrieb über eine Koppelstrecke zur Übertragung der Datensignale gekoppelt ist,
- die vierte Elektrode mit dem elektrischen Massekörper elektrisch verbunden ist und
- die erste und die zweite Koppelstrecke je wenigstens eine kapazitive Verbindung über ein elektrisches Feld umfassen,
**dadurch gekennzeichnet, dass** das elektronische Kommunikationssystem für ein Fahrzeug ausgebildet ist, die Basisstation im Fahrzeug angeordnet ist, der elektrische Massekörper ein Massekörper des Fahrzeugs ist und dass das elektronische Kommunikationssystem wenigstens eine zusätzliche Daten- und/oder Energieübertragungsstrecke zwischen dem (den) Datenträger(n) und der Basisstation enthält.

2. Elektronisches Kommunikationssystem nach Anspruch 1, **dadurch gekennzeichnet, dass** die wenigstens eine zusätzliche Daten- und/oder Energieübertragungsstrecke eine im wesentlichen magnetische Kopplung zwischen dem (den) Datenträger(n) und der Basisstation beinhaltet.

3. Elektronisches Kommunikationssystem nach Anspruch 1, **dadurch gekennzeichnet, dass** die wenigstens eine zusätzliche Daten- und/oder Energieübertragungsstrecke eine im wesentlichen durch elektromagnetische Wellen im UHF-Bereich gebildete Kopplung zwischen dem (den) Datenträger(n) und der Basisstation beinhaltet.

4. Elektronisches Kommunikationssystem nach Anspruch 1, **dadurch gekennzeichnet, dass** die wenigstens eine zusätzliche Daten- und/oder Energieübertragungsstrecke eine im wesentlichen durch infrarotes Licht gebildete Kopplung zwischen dem (den) Datenträger(n) und der Basisstation beinhaltet.

5. Elektronisches Kommunikationssystem nach einem der vorhergehenden Ansprüche **dadurch gekennzeichnet, dass** die erste Koppelstrecke wenigstens teilweise durch den Verschiebungsströme führenden Körper eines Benutzers gebildet ist.

6. Elektronisches Kommunikationssystem nach einem der vorhergehenden Ansprüche **dadurch gekennzeichnet, dass** die zweite Koppelstrecke wenigstens teilweise durch den Boden gebildet ist.

## Claims

1. An electronic communications system, including a base station and at least one portable data carrier which is arranged to exchange data signals with the base station, wherein
- the data carrier includes a first and a second electrode as well as a first data signal processing circuit which is arranged to receive and/or transmit the data signals from and to the base station, respectively, the data signals being formed by a voltage between the first and the second electrode,
- the base station includes at least a third and a fourth electrode as well as a second data signal processing circuit which is arranged to receive and/or transmit the data signals from and to the data carrier (carriers), respectively, the data signals being formed by a voltage between the third and the fourth electrode,
- during operation the second and the third electrode are coupled to each other via a first coupling link for the transmission of the data signals,
- during operation the first electrode is coupled to electrical ground via a second coupling link for the transmission of the data signals,
- the fourth electrode is electrically connected to electrical ground, and
- the first and the second coupling link include each at least one capacitive connection via an electrical field, **characterized in that** the electronic communications system is arranged for a vehicle, the base station is provided in the vehicle, the electrical ground is the ground of the vehicle and **in that** the electronic communications system comprises at least one additional data and/or energy transmission link between the data carrier (carriers) and the base station.

2. An electronic communications system as claimed in claim 1, **characterized in that** the at least one additional data and/or energy transmission link comprises an essentially magnetic coupling between the data carrier (carriers) and the base station.

3. An electronic communications system as claimed in claim 1, **characterized in that** the at least one additional data and/or energy transmission link comprises a coupling between the data carrier (carriers) and the base station, which coupling is formed essentially by electromagnetic waves in the UHF range.

4. An electronic communications system as claimed in claim 1, **characterized in that** the at least one additional data and/or energy transmission link comprises a coupling between the data carrier (carriers) and the base station, which coupling is formed essentially by infrared light.

5. An electronic communications system as claimed in one of the preceding claims, **characterized in that** the first coupling link is formed at least partly by the body of a user which conducts displacement currents.

6. An electronic communications system as claimed in one of the preceding claims, **characterized in that** the second coupling link is formed at least partly by the ground.

## Revendications

1. Système de communication électronique avec une station de base et au moins un support de données mobile qui est conçu pour l'échange de signaux de données avec la station de base dans lequel
- le support de données présente des première et deuxième électrodes ainsi qu'un premier circuit de traitement de signaux de données qui est conçu pour la réception et/ou la transmission des signaux de données de ou vers la station de base, les signaux de données étant formés par une tension entre les première et deuxième électrodes,
- la station de base présente au moins des troisième ou quatrième électrodes ainsi qu'un deuxième circuit de traitement de signaux de données qui sont conçus pour la réception et/ou la transmission de signaux de donnés de ou vers le ou les supports de données, les signaux de données étant formés par une tension entre les troisième et quatrième électrodes,
- les deuxième et troisième électrodes en service sont couplées l'une à l'autre par l'intermédiaire d'une première voie de couplage pour la transmission des signaux de données,
- la première électrode est couplée avec un corps de masse électrique en service par l'intermédiaire d'une voie de couplage pour la transmission des signaux de données,
- la quatrième électrode est reliée électriquement au corps de masse électrique,
- les première et deuxième voies de couplage comprennent chacune au moins une connexion capacitive par l'intermédiaire d'un champ électrique, **caractérisé en ce que** le système de communication électronique est conçu pour un véhicule, la station de base est disposée dans le véhicule, le corps de masse électrique est un corps de masse du véhicule et le système de communication électronique contient au moins une voie supplémentaire de transmission de données et/ou d'énergie entre le ou les supports de données et la station de base.

2. Système de communication électronique selon la revendication 1, **caractérisé en ce que** la voie de transmission de données et/ou d'énergie supplémentaire au moins contient un couplage essentiellement magnétique entre le ou les supports de données et la station de base.

3. Système de communication électronique selon la revendication 1, **caractérisé en ce que** la voie de transmission de donnés et/ou d'énergie supplémentaire au moins contient un couplage essentiellement formé par des ondes électromagnétiques dans la gamme UHF entre le ou les supports de données et la station de base.

4. Système de communication électronique selon la revendication 1, **caractérisé en ce que** la voie de transmission de données et/ou d'énergie supplémentaire au moins contient un couplage essentiellement formé par une lumière infra-rouges entre le ou les supports de données et la station de base.

5. Système de communication électronique selon l'une des revendications précédentes, **caractérisé en ce que** la première voie de couplage est formée, du moins en partie, par le corps d'un utilisateur conduisant les courants de déplacement.

6. Système de communication électronique selon l'une des revendications précédentes, **caractérisé en ce que** la deuxième voie de couplage est formée par le sol, du moins en partie.
